(19) Europäisches Patentamt
European Patent Office
Office européen des brevets

(11) EP 1 524 261 B1

(12) # EUROPÄISCHE PATENTSCHRIFT

(45) Veröffentlichungstag und Bekanntmachung des Hinweises auf die Patenterteilung:
**11.06.2008 Patentblatt 2008/24**

(51) Int Cl.:
*C07C 245/08* (2006.01) *C07C 211/52* (2006.01)

(21) Anmeldenummer: **04023991.5**

(22) Anmeldetag: **08.10.2004**

(54) **Verfahren zur Herstellung von fluorhaltigen Anilinen**

Process for preparing fluorinated anilines

Procédé pour la préparation de derivés fluorés d'aniline

(84) Benannte Vertragsstaaten:
**AT BE BG CH CY CZ DE DK EE ES FI FR GB GR HU IE IT LI LU MC NL PL PT RO SE SI SK TR**

(30) Priorität: **15.10.2003 DE 10347932**

(43) Veröffentlichungstag der Anmeldung:
**20.04.2005 Patentblatt 2005/16**

(73) Patentinhaber: **Saltigo GmbH**
**40764 Langenfeld (DE)**

(72) Erfinder:
- **Mikulas, Mark, Dr.**
  **51375 Leverkusen (DE)**
- **Marhold, Albrecht, Dr.**
  **51373 Leverkusen (DE)**

(74) Vertreter: **Wichmann, Birgid et al**
**LANXESS Deutschland GmbH**
**LIP-IPR**
**Q 18 / 1450**
**51369 Leverkusen (DE)**

(56) Entgegenhaltungen:

- **DATABASE BEILSTEIN BEILSTEIN INSTITUT ZUR FÖRDERUNG DER CHEMISCHEN WISSENSCHAFT, FRANKFURT AM MAIN, DE; XP002318170 gefunden im XFIRE Database accession no. 980172, 3770516 & FARMACO ED. SCI., Bd. 39, Nr. 5, 1984, Seiten 403-413,**
- **DATABASE BEILSTEIN BEILSTEIN INSTITUT ZUR FÖRDERUNG DER CHEMISCHEN WISSENSCHAFT, FRANKFURT AM MAIN, DE; XP002318171 gefunden im XFIRE Database accession no. 3631130, 3751924 & J. MED. CHEM., Bd. 33, Nr. 7, 1990, Seiten 2019-2024,**
- **ERICH BAER AND ANTHONY L. TOSONI: "Formation of Symmetric Azo-compounds from Primary Aromatic Amines by Lead Tetraacetate" J. AM. CHEM. SOC., Bd. 78, 1956, Seiten 2857-2858, XP002318169**
- **DATABASE BEILSTEIN BEILSTEIN INSTITUT ZUR FÖRDERUNG DER CHEMISCHEN WISSENSCHAFT, FRANKFURT AM MAIN, DE; XP002318172 gefunden im XFIRE Database accession no. 4062896 & J. CHEM. SOC., 1965, Seiten 2621-2627,**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).

EP 1 524 261 B1

Printed by Jouve, 75001 PARIS (FR)

## Beschreibung

Die Erfindung betrifft ein Verfahren zur Herstellung von fluorhaltigen Anilinen ausgehend von den entsprechenden chlorhaltigen Anilinen, sowie die Verwendung der fluorhaltigen Aniline.

Fluorhaltige Aniline sind wichtige Zwischenprodukte zur Herstellung von pharmazeutischen Wirkstoffen und Agrochemikalien wie sie beispielsweise in WO-A 98/46607 und WO 98/46608 genannt sind.

Bekannte Verfahren zu ihrer Herstellung umfassen beispielsweise die Nitrierung von fluorhaltigen Aromaten und die anschließende Reduktion der Nitroverbindungen zu den entsprechenden Anilinen. Auf diese Weise können beispielsweise 4-Fluoranilin und 2,4-Difluoranilin aus Fluorbenzol bzw. 1,3-Difluorbenzol erhalten werden (siehe J. Org. Chem., 1962, 27, 1910 - 11; J. Fluor. Chem., 2002, 113 (2), 207 - 9). Nachteilig an diesem Verfahren ist, dass die fluorierten Aromaten sehr teuer sind und die Nitrierung nur ein begrenztes Substitutionsmuster am aromatischen Kern erlaubt.

Ein weiteres Verfahren zur Herstellung von fluorhaltigen Anilinen besteht im Hofmann-Abbau von fluorhaltigen Benzoesäureamiden. Auf diese Weise läßt sich 2,6-Difluoranilin herstellen, das auf vorstehend beschriebenem Weg nicht zugänglich ist. Allerdings sind die Ausbeuten für diesen Abbau nur mäßig, weshalb diese Methode für die industrielle Anwendung nicht in Betracht kommt.

Ein weiteres Verfahren zur Herstellung fluorhaltiger Aniline verläuft ausgehend von Chlornitrobenzolen über eine Chlor-Fluor-Austauschreaktion (Halex-Reaktion) mit anschließender Hydrierung direkt zu den gewünschten Produkten. Die Nitrogruppe aktiviert Chloratome in 2-, 4- und 6-Stellung, so daß auf diese Weise beispielsweise aus 2,4-Dichlornitrobenzol nach Halex-Reaktion und Reduktion 2,4-Difluoranilin erhalten wird (siehe Tetrahedron, 1995, 51, 6363). Bei höher chlorierten Nitrobenzolen oder bei Anwesenheit von Substituenten, die die Nitrogruppe für die Halex-Reaktion aktivieren, versagt dieses Verfahren jedoch in der Regel (siehe Tetrahedron, 1999, 55, 7725 - 7738; J. Am. Chem. Soc. 1956, 78, 6034).

Es besteht deshalb weiterhin Bedarf an einem Verfahren zur Herstellung von fluorhaltigen Anilinen, das die vorstehend beschriebenen Nachteile überwindet und einen einfachen Zugang zu fluorhaltigen Anilinen ermöglicht.

Es wurde nun ein Verfahren zur Herstellung von Verbindungen der Formel (I) gefunden,

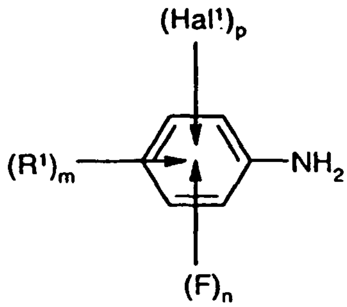

(I)

in der

m für eine ganze Zahl zwischen 0 und (5-n-p) steht

n für 1, 2, 3 oder 4 steht und

p für 0,1 oder 2 steht,

wobei die Summe aus

n+p maximal 4 ist und

$R^1$ jeweils unabhängig für Wasserstoff, Fluor, $C_1$-$C_{12}$-Alkyl, $C_5$-$C_{14}$-Aryl, $C_6$-$C_{15}$-Arylalkyl, $C_1$-$C_{12}$-Fluoralkyl, $C_1$-$C_{12}$-Fluoralkylthio, $C_1$-$C_{12}$-Fluoralkoxy, $C_1$-$C_{12}$-Alkylsulfonyl oder Reste der Formeln (IIa) bis (IIf) steht

A-CN (IIa) A-CON($R^2$)$_2$- (IIb)

(fortgesetzt)

| | | | |
|---|---|---|---|
| A-$CO_2R^2$ | (IIc) | A-$CONH_2$ | (IId) |
| A-$COHal^2$ | (IIe) | A-B-$R^2$ | (IIf) |

wobei in den Formeln (IIa) bis (IIf)

A fehlt oder für einen $C_1$-$C_8$-Alkylen- oder $C_1$-$C_8$-Fluoralkylenrest steht und

B für Sauerstoff oder $NR^2$ steht, und

$R^2$ jeweils unabhängig für $C_1$-$C_8$-Alkyl, $C_6$-$C_{15}$-Arylalkyl oder $C_5$-$C_{14}$-Aryl steht oder gegebenenfalls $N(R^2)_2$ als Ganzes für einen cyclischen Aminorest mit insgesamt 4 bis 12 Kohlenstoffatomen steht und

$Hal^1$ für Chlor oder Brom steht,

das dadurch gekennzeichnet ist, dass

- in einem Schritt A)
  Verbindungen der Formel (III)

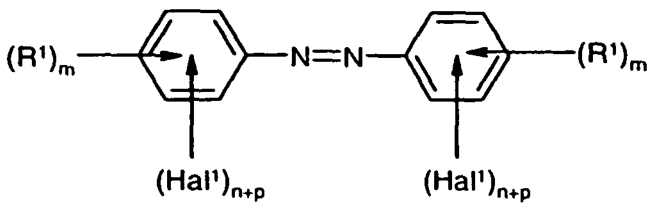

(III)

in der $R^1$, $Hal^1$, m, p und n die vorstehend genannte Bedeutung besitzen
in Gegenwart von ionischem Fluorid in Verbindungen der Formel (IV) überführt werden

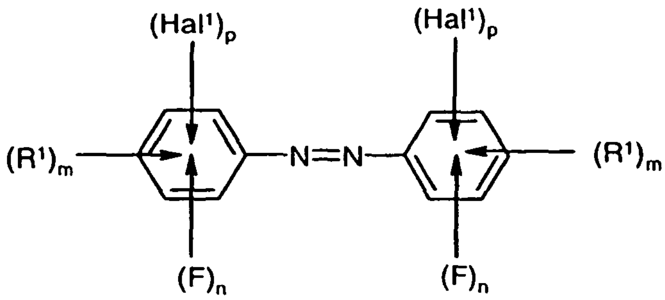

(IV)

und

- in einem Schritt B)
  die Verbindungen der Formel (IV) mit einem Reduktionsmittel in die Verbindungen der Formel (I) überführt werden.

Im Rahmen der Erfindung können alle oben stehenden und im Folgenden aufgeführten, allgemeinen oder in Vorzugsbereichen genannten Restedefinitionen, Parameter und Erläuterungen untereinander, also auch zwischen den jeweiligen Bereichen und Vorzugsbereichen in beliebiger Weise kombiniert werden.
**Alkyl** beziehungsweise **Alkylen** steht jeweils unabhängig für einen geradkettigen, cyclischen, verzweigten oder unverzweigten Alkyl- beziehungsweise Alkylen-Rest. Gleiches gilt für den nichtaromatischen Teil eines Arylalkyl-Restes.

$C_1$-$C_4$-Alkyl steht beispielsweise für Methyl, Ethyl, n-Propyl, iso-Propyl, n-Butyl, sec.-Butyl und tert.-Butyl, $C_1$-$C_{12}$-Alkyl darüber hinaus beispielsweise für n-Pentyl, 1-Methylbutyl, 2-Methylbutyl, 3-Methylbutyl, neo-Pentyl, 1-Ethylpropyl, cyclo-Hexyl, cyclo-Pentyl, n-Hexyl, n-Heptyl, n-Octyl, n-Nonyl, n-Decyl und n-Dodecyl.

$C_1$-$C_8$-Alkylen steht beispielsweise für Methylen, 1,1-Ethylen, 1,2-Ethylen, 1,1-Propylen, 1,3-Propylen, 1,4-Butylen, 1,2-cyclo-Hexoxylen und 1,2-cyclo-Pentylen.

$C_2$-$C_8$-Alkenylen steht beispielsweise für 1,1-Ethenylen 2-Ethoxy-1,1-ethenylen und 2-Methoxy-1,1-ethenylen.

**Fluoralkyl** beziehungsweise **Fluoralkylen** bedeutet jeweils unabhängig einen geradkettigen, cyclischen, verzweigten oder unverzweigten Alkyl-Rest beziehungsweise Alkylen-Rest, der einfach, mehrfach oder vollständig durch Fluoratome substituiert ist.

Beispielsweise steht $C_1$-$C_{12}$-Fluoralkyl für Trifluormethyl, 2,2,2-Trifluorethyl, Pentafluorethyl, Nonafluorbutyl, Heptafluorisopropyl, Perfluoroctyl und Perfluordodecyl.

**Aryl** steht jeweils unabhängig für einen heteroaromatischen Rest mit 5 bis 14 Gerüstkohlenstoffatomen, in denen keines, ein, zwei oder drei Gerüstkohlenstoffatome pro Cyclus, im gesamten Molekül mindestens jedoch ein Gerüstkohlenstoffatom, durch Heteroatome, ausgewählt aus der Gruppe Stickstoff, Schwefel oder Sauerstoff, substituiert sein können, oder und vorzugsweise für einen carbocyclischen aromatischen Rest mit 6 bis 14 Gerüstkohlenstoffatomen.

Weiterhin kann der carbocyclische aromatische Rest oder heteroaromatische Rest mit bis zu fünf gleichen oder verschiedenen Substituenten pro Cyclus substituiert sein, die ausgewählt sind aus der Gruppe Fluor, Cyano, Nitro, $C_1$-$C_{12}$-Alkyl, $C_1$-$C_{12}$-Fluoralkyl, $C_1$-$C_{12}$-Fluoralkoxy, $C_1$-$C_{12}$-Fluoralkylthio, $C_1$-$C_{12}$-Alkoxy, Di($C_1$-$C_8$-alkyl)amino oder Resten der Formeln (IIa) bis (IIf) mit der vorstehend genannten Bedeutung.

**Arylalkyl** bedeutet jeweils unabhängig einen geradkettigen, cyclischen, verzweigten oder unverzweigten Alkyl-Rest nach vorstehender Definition, der einfach, mehrfach oder vollständig durch Aryl-Reste gemäß vorstehender Definition substituiert sein kann.

Im Folgenden werden die bevorzugten Substitutionsmuster definiert:

n steht bevorzugt für 1, 2 oder 3, besonders bevorzugt für 3,

$R^1$ steht bevorzugt für Wasserstoff, $C_1$-$C_4$-Alkyl, Trifluormethyl, Halogencarbonyl, oder Cyano, besonders bevorzugt für Wasserstoff.

In Verbindungen der Formel (I) steht p vorzugsweise für 0.

Das erfindungsgemäße Verfahren eignet sich insbesondere zur Herstellung von 2,4,6-Trifluoranilin, 2-Trifluormethyl-4-fluoranilin, 4-Trifluormethyl-2-fluoranilin, 4-Trifluormethyl-2,6-difluoranilin und 3-Trifluormethyl-2,4,6-trifluoranilin.

Die als Edukte verwendeten Verbindungen der Formel (III) werden vorzugsweise so hergestellt, dass in einem ersten Schritt die Halogenierung, vorzugsweise Chlorierung von Verbindungen der Formel (V) zu Verbindungen der Formel (VI) erfolgt, wobei in den Formeln (V) und (VI)

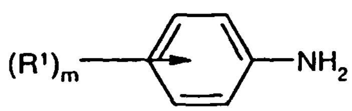

(V)

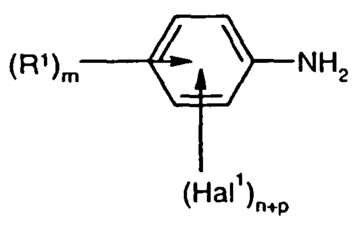

(VI)

$R^1$ , $Hal^1$, n, p und m die oben stehend angegebenen Bedeutungen besitzen
und in einem zweiten Schritt die Oxidation der Verbindungen der Formel (VI) zu den Verbindungen der Formel (III) erfolgt.

Bevorzugte Verbindungen der Formel (VI) sind 2,4,6-Trichloranilin, 2-Trifluormethyl-4-chloranilin, 4-Trifluormethyl-2-chloranilin, 4-Trifluormethyl-2,6-dichloranilin und 3-Trifluormethyl-2,4,6-trichloranilin.

Einige Verbindungen der Formel (III) sind bislang unbekannt, jedoch über das vorstehend beschrieben Verfahren zugänglich. Daher sind von der Erfindung auch 2,2'-Dichlor-4,4'-bistrifluormethylazobenzol, 2,2',6,6'-Tetrachlor-4,4'-bistrifluormethyl-azobenzol, 2,2',4,4',6,6'-Hexachlor-3,3'-bistrifluormethyl-azobenzol und 4,4'-Dichlor-2,2'-bistrifluormethylazobenzol umfasst.

Einige Verbindungen der Formel (IV), die unverzichtbare Intermediate für das erfindungsgemäße Verfahren sind, sind ebenfalls bislang unbekannt. Daher sind von der Erfindung auch 2,2'-Difluor-4,4'-bistrifluormethylazobenzol, 2,2',6,6'-Tetrafluor-4,4'-bistrifluormethylazobenzol, 2,2',4,4',6,6'-Hexafluor-3,3'-bistrifluormethylazobenzol und 4,4'-Difluor-2,2'-bistrifluormethylazobenzol umfasst.

Sowohl die Halogenierung von Anilinen als auch die Oxidation von Anilinen oder deren Salzen zu den entsprechenden Diazobenzolen sind prinzipiell z.B. aus Bull. Soc. Chim. Belg., 1993, 102 (1), 59 - 62; Org. Synth., 1960, 40, 18) bekannt und sind auf die Verbindungen der Formeln (V) und (VI) anwendbar.

Gemäß Schritt A) des erfindungsgemäßen Verfahrens werden Verbindungen der Formel (III) in Gegenwart von ionischen Fluorid in Verbindungen der Formel (IV) überführt.

Ionische Fluoride sind beispielsweise quartäre Ammonium- oder Phosphoniumfluoride sowie Alkalimetallfluoride oder Mischungen der genannten Verbindungen.

Beispiele für Ammonium- oder Phosphoniumfluoride sind solche der Formel (VI),

$$(\text{Kation}^+)(\text{F}^-) \qquad \text{(VI)}$$

in der (Kation$^+$) für substituierte quartäre Ammonium oder Phosphonium-Kationen wie insbesonders Kationen der Formel (VII) steht,

$$[\text{Pnyc}(C_1\text{-}C_{12}\text{-Alkyl})_q(C_6\text{-}C_{15}\text{-Arylalkyl})_r(C_5\text{-}C_{14}\text{-Aryl})_s(\{(C_2\text{-}C_6\text{-Alkyl})\text{-O}]_v\text{-}(C_1\text{-}C_8\text{-Alkyl})\}_t)]^+ \qquad \text{(VII)}$$

in der Pnyc für Stickstoff oder Phosphor steht und
in denen jeweils (q+r+s+t) = 4 ist.

Bevorzugt werden im Schritt A) Alkalimetallfluoride wie insbesondere Natrium-, Kalium- und Cäsiumfluorid oder Mischungen davon eingesetzt. Besonders bevorzugt ist Kaliumfluorid.

Das molare Verhältnis von ionischem Fluorid zu auszutauschenden Brom- oder Chloratomen in Verbindungen der Formel (III) kann beispielsweise 0,7 bis 5 betragen, vorzugsweise 0,9 bis 2 und besonders bevorzugt 1,2 bis 1,5. Die Menge an ionischem Fluorid ist nach oben prinzipiell nicht begrenzt, größere Mengen sind aber unwirtschaftlich.

Vorzugsweise wird Schritt A) weiterhin in Gegenwart von Phasentransferkatalysatoren und/oder Halex-Katalysatoren durchgeführt.

Als Phasentransferkatalysatoren sind beispielsweise Kronenether, wie 18-Krone-6, 12-Krone-4, Dibenzo-18-Krone-6 oder Dibenzo-12-Krone-4, Kryptanden wie Kryptand[2.2.2] oder Podanden wie Polyglykolether oder solche der Formel (VIII) geeignet,

$$(\text{Kation}^+)(\text{Anion}^-) \qquad \text{(VIII)}$$

in der

(Kation$^+$) die unter der Formel (VI) angegebene Bedeutung einschließlich deren Vorzugsbereiche besitzt und

(Anion$^-$) für das Anion einer organischen oder anorganischen Säure steht, wobei Chlorid, Bromid, Iodid, Acetat, Nitrat, Sulfat, Hydrogensulfat, Tetrafluoroborat, Hexafluorophosphat, Tosylat, und Triflat bevorzugt und Chlorid, Bromid, Iodid, Sulfat und Hydrogensulfat besonders bevorzugt sind.

Halex-Katalysatoren sind beispielsweise Tetrakis(dialkylamino)phosphoniumverbindungen (WO 98/05610) oder solche, die in EP-A 1 266 904 beschrieben werden.

Vorzugsweise wird Schritt A) weiterhin in Gegenwart von organischem Lösungsmittel durchgeführt. Als organische Lösungsmittel sind beispielsweise geeignet: Ketone, wie Aceton, 2-Butanon oder Methyl-isobutyl-keton; Nitrile

wie beispielsweise Acetonitril, Propanitril, Benzonitril, Benzylnitril oder Butyronitril; Amide wie beispielsweise N,N-Dimethylformamid, N,N-Dimethylimidazolinon, N,N-Dimethylacetamid, N-Methyl-formanilid, N-Methyl-pyrrolidon, N-Methylcaprolactam oder Hexamethylphosphorsäuretriamid; Sulfoxide wie beispielsweise Dimethylsulfoxid, Sulfone wie beispielsweise Tetramethylensulfon, Polyether wie beispielsweise 1,4-Dioxan, Ethylenglykoldimethylether, Ethylenglykoldiethylether, Diethylenglykoldimethylether oder Diethylenglykoldiethylether oder Gemische solcher organischen Lösungsmittel. Bevorzugte Lösungsmittel sind: N,N-Dimethylformamid, N,N-Dimethylimidazolinon, N-Methyl-pyrrolidon, Dimethylsulfoxid, und Tetramethylensulfon.

Vorzugsweise beträgt der Wassergehalt des Lösungsmittels maximal 1 Gew-%, bevorzugt maximal 0,2 Gew-% und besonders bevorzugt 0,05 Gew.-%. Vorzugsweise wird ein solcher Wassergehalt durch Andestillieren oder Trocknung in an sich bekannter Weise erreicht. Bei Einsatz von Alkalimetallfluoriden wird besonders bevorzugt das Lösungsmittel gleichzeitig in Gegenwart des eingesetzten Alkalimetallfluorids getrocknet bzw. andestilliert.

Die Reaktionstemperatur in Schritt A) kann beispielsweise 120°C bis 250°C, vorzugsweise 160 bis 220°C betragen.

Der Reaktionsdruck kann beispielsweise 0,5 bis 100 bar betragen, Umgebungsdruck ist bevorzugt.

Die Reaktionsdauer kann beispielsweise 10 min bis 72 Stunden, bevorzugt 2 bis 20 Stunden betragen.

Schritt B) kann in Analogie zu prinzipiell bekannten Verfahren beispielsweise durch Hydrogenolyse der Verbindungen der Formel (IV) mit einem Wasserstoffdonor in Gegenwart von Übergangsmetallkatalysatoren erfolgen. Wasserstoffdonoren sind beispielsweise Hydrazin, Ameisensäure oder Formiate sowie elementarer Wasserstoff selbst. Alternativ kann die Reduktion auch mit Zink und Ammoniumformiat gemäß Tetrahedron Letters, 2002, 43,7, 1329 -1331 erfolgen.

Das erfindungsgemäße Verfahren zur Herstellung von fluorhaltigen Anilinen zeichnet sich dadurch aus, dass die Einsatzmaterialien sehr einfach und preiswert beispielsweise durch Chlorierung von Anilinen und anschließende Oxidation zu den Azobenzolen hergestellt werden können und die Synthese in hohen Ausbeuten zu den gewünschten Produkten verläuft. Es ist als überraschend einzuschätzen, dass die Halex-Reaktion unter den genannten Bedingungen überhaupt und ohne Bildung nennenswerter Nebenprodukte stattfindet.

Die erfindungsgemäß herstellbaren Verbindungen der Formel (I) eignen sich insbesondere in einem Verfahren zur Herstellung von Arzneimitteln und Agrochemikalien, wie sie zum Beispiel in WO-A 98/46607 und WO 98/46608 genannt sind.

## Beispiele:

### Beispiel 1

#### Herstellung von 2,2',4,4',6,6'-Hexafluorazobenzol

Unter Feuchtigkeitsausschluß werden in 51 g Sulfolan, 4,3 g getrocknetes Kaliumfluorid und 3,0 g 2,2',4,4',6,6'-Hexachlorazobenzol vorgelegt und 0,2 g Tetraphenylphosphoniumbromid zugegeben. Anschließend wird auf 180°C erhitzt und für 12 Stunden gerührt. Nach Abkühlen wird auf 150 ml Wasser ausgetragen und der Ansatz zweimal mit je 50 ml Cyclohexan extrahiert. Man erhält nach Kristallisation 1,7 g = 76 % d. Th. Des gewünschten Produktes.

### Beispiel 2

#### Herstellung von 2,4,6-Trifluoranilin

1,0 g 2,2',4,4',6,6'-Hexafluorazobenzol aus Beispiel 1 werden in 20 ml Methanol bei Raumtemperatur gelöst und nacheinander 0,6 g Ammoniumforminat und 0,5 g Zinkpulver zugegeben. Es wird für eine Stunde bei Raumtemperatur nachgerührt. Laut GC-Analyse ist dann das Ausgangsmaterial vollständig umgesetzt. Nach Abdestillieren des Methanols wird das 2.4.6-Trifluoranilin in Cyclohexan aufgenommen, getrocknet und das Lösungsmittel erneut abdestilliert. Es verbleiben 0,9 g des gewünschten Produktes (89 % d. Th.).

### Beispiel 3

#### Herstellung von 2.2'-Bistrifluormethyl-4,4'-difluorazobenzol

Analog Beispiel 1 werden 9,8 g 2.2'-Bistrifluormethyl-4,4'-dichlorazobenzol, 4 g Kaliumfluorid, 0,5 g Tetraphenylphosphoniumbromid in 50 ml Sulfolan bei 175°C für 15 Stunden zur Reaktion gebracht. Es werden nach Kristallisation 5,8 g 2.2'-Bistrifluormethyl-4.4'-difluorazobenzol (65 % d.Th.) erhalten.

**Beispiel 4**

**Herstellung von 2-Trifluormethyl-4-fluoranilin**

5 g des Produktes aus Beispiel 3 werden in 60 ml Toluol gelöst und mit 0,2 g 5%igem Palladium auf Kohle versetzt. Anschließend wird für 5 Stunden bei 60°C und 20 bar Wasserstoff hydriert. Die Umsetzung zum gewünschten Produkt erfolgte quantitativ.

## Patentansprüche

1. Verfahren zur Herstellung von Verbindungen der Formel (I),

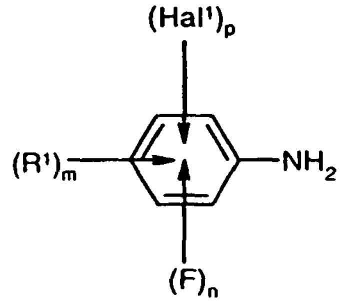

(I)

in der

m für eine ganze Zahl zwischen 0 und (5-n-p) steht
n für 1, 2, 3 oder 4 steht und
p für 0,1 oder 2 steht,

wobei die Summe aus

n+p maximal 4 ist und
$R^1$ jeweils unabhängig für Wasserstoff, Fluor, $C_1$-$C_{12}$-Alkyl, $C_5$-$C_{14}$-Aryl, $C_6$-$C_{15}$Arylalkyl, $C_1$-$C_{12}$-Fluoralkyl, $C_1$-$C_{12}$-Fluoralkylthio, $C_1$-$C_{12}$-Fluoralkoxy, $C_1$-$C_{12}$-Alkylsulfonyl oder Reste der Formeln (IIa) bis (IIf) steht

A-CN (IIa)

A-CON($R^2$)$_2$- (IIb)

A-$CO_2R^2$ (IIc)

A-$CONH_2$ (IId)

A-COHal$^2$ (IIe)

A-B-$R^2$ (IIf)

wobei in den Formeln (IIa) bis (110

A fehlt oder für einen $C_1$-$C_8$-Alkylen- oder $C_1$-$C_8$-Fluoralkylenrest steht und
B für Sauerstoff oder $NR^2$ steht, und
$R^2$ jeweils unabhängig für $C_1$-$C_8$-Alkyl, $C_6$-$C_{15}$-Arylalkyl oder $C_5$-$C_{14}$-Aryl steht oder gegebenenfalls N($R^2$)$_2$ als Ganzes für einen cyclischen Aminorest mit insgesamt 4 bis 12 Kohlenstoffatomen steht und
Hal$^1$ für Chlor oder Brom steht,

**dadurch gekennzeichnet, dass**

- in einem Schritt A)

Verbindungen der Formel (III)

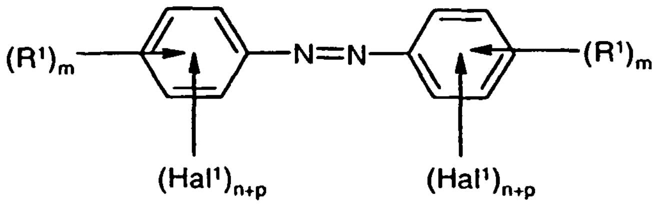

(III)

in der

$R^1$, $Hal^1$, m, p und n die vorstehend genannte Bedeutung besitzen und

in Gegenwart von ionischem Fluorid in Verbindungen der Formel (IV) überführt werden

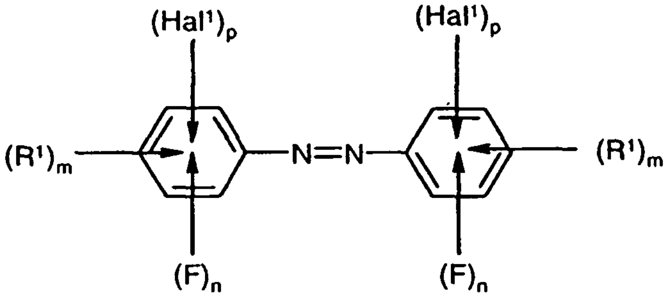

(IV)

und

- in einem Schritt B)

die Verbindungen der Formel (IV) mit einem Reduktionsmittel in die Verbindungen der Formel (I) überführt werden.

**2.** Verfahren nach Anspruch 1, **dadurch gekennzeichnet, dass** als Verbindungen der Formel (I) 2,4,6-Trifluoranilin, 2-Trifluormethyl-4-fluoranilin, 4-Trifluormethyl-2-fluoranilin, 4-Trifluormethyl-2,6-difluoranilin und 3-Trifluormethyl-2,4,6-trifluoranilin hergestellt werden.

**3.** Verfahren nach einem oder mehreren der Ansprüche 1 und 2, **dadurch gekennzeichnet, dass** die als Edukte verwendeten Verbindungen der Formel (III) so hergestellt werden, dass in einem ersten Schritt die Halogenierung, vorzugsweise Chlorierung von Verbindungen der Formel (V) zu Verbindungen der Formel (VI) erfolgt, wobei in den Formeln (V) und (VI)

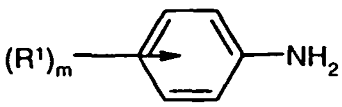

(V)

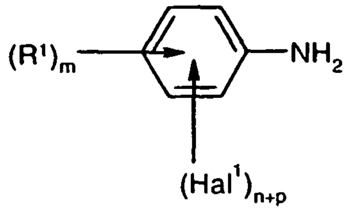

(VI)

$R^1$ , $Hal^1$, n, p und m die in Anspruch 1 angegebenen Bedeutungen besitzen und in einem anschließenden Schritt die Oxidation der Verbindungen der Formel (VI) zu den Verbindungen der Formel (III) erfolgt.

4. Verfahren nach einem oder mehreren der Ansprüche 1 bis 3, **dadurch gekennzeichnet, dass** als ionisches Fluorid quartäre Ammonium- oder Phosphoniumfluoride sowie Alkalimetallfluoride oder Mischungen der genannten Verbindungen eingesetzt werden.

5. Verfahren nach einem oder mehreren der Ansprüche 1 bis 4, **dadurch gekennzeichnet, dass** Schritt A) weiterhin in Gegenwart von Phasentransferkatalysatoren und/oder Halex-Katalysatoren durchgeführt wird.

6. Verfahren nach einem oder mehreren der Ansprüche 1 bis 5, **dadurch gekennzeichnet, dass** Schritt A) in Gegenwart von organischem Lösungsmittel durchgeführt wird.

7. Verfahren nach einem oder mehreren der Ansprüche 1 bis 6, **dadurch gekennzeichnet, dass** in Schritt A) die Reaktionstemperatur in Schritt A) 120°C bis 250°C beträgt.

8. 2,2'-Dichlor-4,4'-bistrifluormethylazobenzol, 2,2',6,6'-Tetrachlor-4,4'-bistrifluormethylazobenzol, 2,2',4,4',6,6'-Hexachlor-3,3'-bistrifluormethyl-azobenzol und 4,4'-Dichlor-2;2'-bistrifluormethylazobenzol.

9. 2,2'-Difluor-4,4'-bistrifluormethylazobenzol, 2,2',6,6'-Tetrafluor-4,4'-bistrifluormethylazobenzol, 2,2',4,4',6,6'-Hexafluor-3,3'-bistrifluormethylazobenzol und 4,4'-Difluor-2,2'-bistrifluormethylazobenzol.

**Claims**

1. Process for preparing compounds of the formula (I)

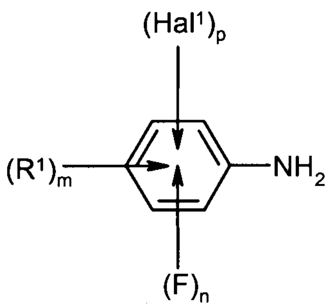

(I)

in which

m is an integer in the range from 0 to (5-n-p)
n is 1, 2, 3 or 4 and
p is 0, 1 or 2,

where the sum of
n+pis a maximum of 4 and

$R^1$ is in each case independently hydrogen, fluorine, $C_1$-$C_{12}$-alkyl, $C_5$-$C_{14}$-aryl, $C_6$-$C_{15}$-arylalkyl, $C_1$-$C_{12}$-fluoroalkyl, $C_1$-$C_{12}$-fluoroalkylthio, $C_1$-$C_{12}$-fluoroalkoxy, $C_1$-$C_{12}$-alkylsulphonyl or radicals of the formulae (IIa) to (IIf)

A-CN (IIa)

A-CON $(R^2)_2$- (IIb)

A-$CO_2R^2$ (IIc)

A-$CONH_2$ (IId)

A-COHal$^2$ (IIe)

A-B-$R^2$ (IIf)

where, in the formulae (IIa) to (IIf),

A is absent or is a $C_1$-$C_8$-alkylene or $C_1$-$C_8$-fluoroalkylene radical and
B is oxygen or $NR^2$, and
$R^2$ is in each case independently $C_1$-$C_8$-alkyl, $C_6$-$C_{15}$-arylalkyl or $C_5$-$C_{14}$-aryl, or N $(R^2)_2$ as a whole is optionally a cyclic amino radical having a total of 4 to 12 carbon atoms and
Hall is chlorine or bromine,

**characterized in that**,

• in a step A),
compounds of the formula (III)

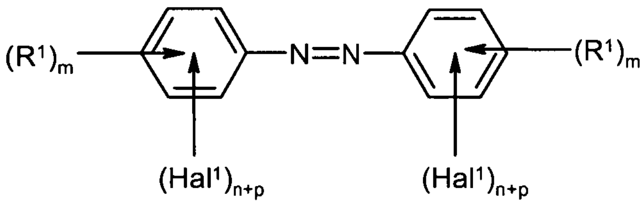

(III)

in which
$R^1$, $Hal^1$, m, p and n are each as defined above
are converted in the presence of ionic fluoride to compounds of the formula (IV)

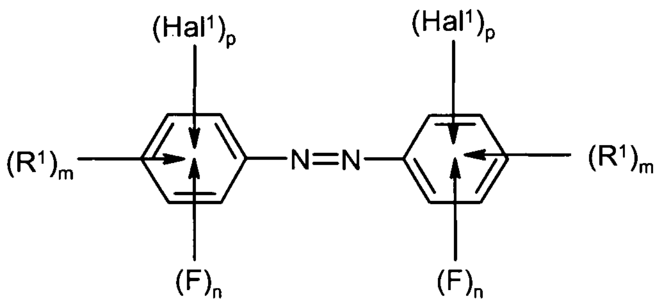

(IV)

and,

• in a step B),

the compounds of the formula (IV) are converted to the compounds of the formula (I) with a reducing agent.

2. Process according to Claim 1, **characterized in that** the compounds of the formula (I) prepared are 2,4,6-trifluoroaniline, 2-trifluoromethyl-4-fluoroaniline, 4-trifluoromethyl-2-fluoroaniline, 4-trifluoromethyl-2,6-difluoroaniline and 3-trifluoromethyl-2,4,6-trifluoroaniline.

3. Process according to either of Claims 1 and 2, **characterized in that** the compounds of the formula (III) which are used as reactants are prepared in such a way that, in a first step, compounds of the formula (V) are halogenated, preferably chlorinated, to give compounds of the formula (VI), where, in the formulae (V) and (VI)

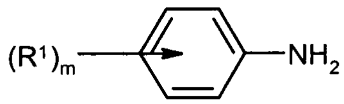

(V)

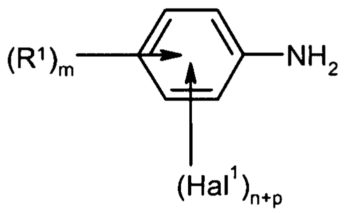

(VI)

$R^1$, $Hal^1$, n, p and m are each as defined in Claim 1 and, in a subsequent step, the compounds of the formula (VI) are oxidized to the compounds of the formula (III).

4. Process according to one or more of Claims 1 to 3, **characterized in that** the ionic fluoride used is a quaternary ammonium fluoride or phosphonium fluoride, or else an alkali metal fluoride or a mixture of the compounds mentioned.

5. Process according to one or more of Claims 1 to 4, **characterized in that** step A) is also carried out in the presence of phase transfer catalysts and/or halex catalysts.

6. Process according to one or more of Claims 1 to 5, **characterized in that** step A) is carried out in the presence of organic solvents.

7. Process according to one or more of Claims 1 to 6, **characterized in that** the reaction temperature in step A) is 120°C to 250°C.

8. 2,2'-Dichloro-4,4'-bistrifluoromethylazobenzene, 2,2',6,6'-tetrachloro-4,4'-bistrifluoromethylazobenzene, 2,2',4,4',6,6'-hexachloro-3,3'-bistrifluoromethylazobenzene and 4,4'-dichloro-2,2'-bistrifluoromethylazobenzene.

9. 2,2'-Difluoro-4,4'-bistrifluoromethylazobenzene, 2,2',6,6'-tetrafluoro-4,4'-bistrifluoromethylazobenzene, 2,2',4,4',6,6'-hexafluoro-3,3'-bistrifluoromethylazobenzene and 4,4'-difluoro-2,2'-bistrifluoromethylazobenzene.

## Revendications

1. Procédé pour la préparation de composés de formule (I) ,

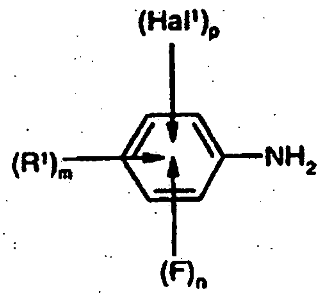

(I)

dans laquelle

m représente un nombre entier compris entre 0 et (5-n-p),
n représente 1, 2, 3 ou 4, et
p représente 0, 1 ou 2,

la somme de

n+p était égale à 4 au maximum et
$R^1$ représentent chacun indépendamment un atome d'hydrogène ou de fluor ou un groupe alkyle en $C_1$-$C_{12}$, aryle en $C_5$-$C_{14}$, arylalkyle en $C_6$-$C_{15}$, fluoroalkyle en $C_1$-$C_{12}$, fluoroalkylthio en $C_1$-$C_{12}$, fluoroalcoxy en $C_1$-$C_{12}$, alkyl ($C_1$-$C_{12}$) -sulfonyle ou des radicaux de formules (IIa) à (IIf)

A-CN (IIa)

A-CON $(R^2)_2$- (IIb)

A-$CO_2R^2$ (IIc)

A-$CONH_2$ (IId)

A-$COHal^2$ (IIe)

A-B-$R^2$ (IIf)

dans les formules (IIa) à (IIf)

A étant absent ou représentant un radical alkylène en $C_1$-$C_8$ ou fluoroalkylène en $C_1$-$C_8$ et
B représentant un atome d'oxygène ou $NR^2$, et
$R^2$ représentant chacun indépendamment un groupe alkyle en $C_1$-$C_8$, arylalkyle en $C_6$-$C_{15}$ ou aryle en $C_5$-$C_{14}$ ou éventuellement $N(R^2)_2$ représentant dans son ensemble un radical amino cyclique ayant au total 4 à 12 atomes de carbone et
$Hal^1$ représentant le chlore ou le brome,

**caractérisé en ce que**

• dans une étape A)
on convertit des composés de formule (III)

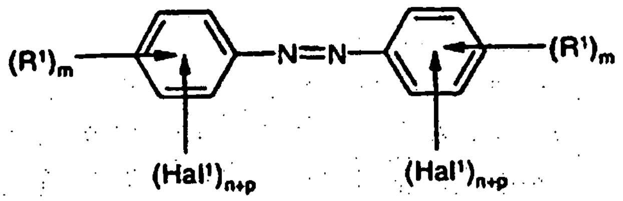

(III)

dans laquelle
$R^1$, $Hal^1$, m, p et n ont les significations données précédemment
en présence de fluorure ionique, en composés de formule (IV)

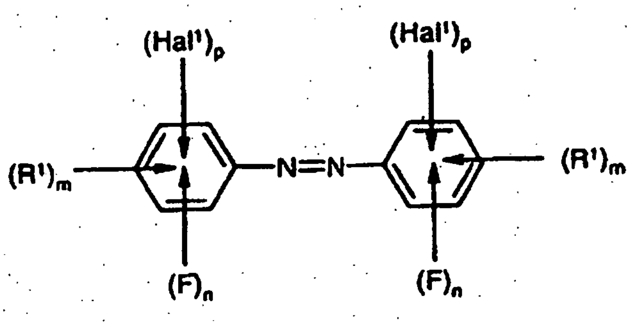

(IV)

et
• dans une étape B)
on convertit les composés de formule (IV), à l'aide d'un réducteur, en les composés de formule (I).

2. Procédé selon la revendication 1, **caractérisé en ce qu'**on prépare en tant que composés de formule (I) la 2,4,6-trifluoroaniline, la 2-trifluorométhyl-4-fluoroaniline, la 4-trifluorométhyl-2-fluoroaniline, le 4-trifluorométhyl-2,6-difluoroaniline et la 3-trifluorométhyl-2,4,6-trifluoroaniline.

3. Procédé selon une ou plusieurs des revendications 1 et 2, **caractérisé en ce qu'**on prépare les composés de formule (III), utilisés comme produits de départ, en effectuant dans une première étape l'halogénation de préférence la chloration, de composés de formule (V) en composés de formule (VI), dans les formules (V) et (VI)

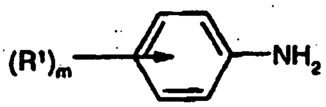

(V)

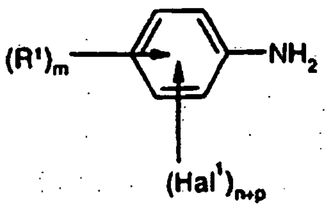

(VI)

$R^1$, $Hal^1$, n, p et m ayant les significations indiquées dans la revendication 1, et en effectuant, dans une étape subséquente, l'oxydation des composés de formule (VI) en les composés de formule (III).

4. Procédé selon une ou plusieurs des revendications 1 à 3, **caractérisé en ce qu'**on utilise en tant que fluorure ionique des fluorures d'ammonium ou phosphonium quaternaire ainsi que des fluorures de métaux alcalins ou des mélanges des composés cités.

5. Procédé selon une ou plusieurs des revendications 1 à 4, **caractérisé en ce que** l'étape A) est en outre effectuée en présence de catalyseurs de transfert de phase et/ou de catalyseurs Halex.

6. Procédé selon une ou plusieurs des revendications 1 à 5, **caractérisé en ce qu'**on effectue l'étape A) en présence d'un solvant organique.

7. Procédé selon une ou plusieurs des revendications 1 à 6, **caractérisé en ce que** dans l'étape A) la température de réaction est de 120 °C à 250 °C.

8. 2,2'-dichloro-4,4'-bistrifluorométhylazobenzène, 2,2',6,6'-tétrachloro-4,4'-bis-trifluorométhylazobenène, 2,2',4,4',6,6'-hexachloro-3,3'-bis-trifluorométhylazobenène et 4,4'-dichloro-2,2'-bis-trifluorométhylazobenzène.

9. 2,2'-difluoro-4,4'-bistrifluorométhylazobenzène, 2,2',6,6'-tétrafluoro-4,4'-bis-trifluorométhylazobenène, 2,2',4,4',6,6'-hexafluoro-3,3'-bis-trifluorométhylazobenène et 4,4'-difluoro-2,2'-bis-trifluorométhylazobenzène.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- WO 9846607 A
- WO 9846608 A
- WO 9805610 A
- EP 1266904 A


### In der Beschreibung aufgeführte Nicht-Patentliteratur


- *J. Org. Chem.,* 1962, vol. 27, 1910-11
- *J. Fluor. Chem.,* 2002, vol. 113 (2), 207-9
- *Tetrahedron,* 1995, vol. 51, 6363
- *Tetrahedron,* 1999, vol. 55, 7725-7738
- *J. Am. Chem. Soc.,* 1956, vol. 78, 6034
- *Bull. Soc. Chim. Belg.,* 1993, vol. 102 (1), 59-62
- *Org. Synth.,* 1960, vol. 40, 18
- *Tetrahedron Letters,* 2002, vol. 43 (7), 1329-1331